# EUROPEAN PATENT SPECIFICATION

(11) **EP 4 237 017 B1**
(45) Date of publication and mention of the grant of the patent: **18.02.2026**
(21) Application number: 21811539.2
(22) Date of filing: 17.11.2021
(51) Int. Cl.: A61L 11/00

(54) **CHAMBER, SYSTEM AND METHOD FOR PROCESSING MEDICAL WASTE**
KAMMER, SYSTEM UND VERFAHREN ZUR VERARBEITUNG VON MEDIZINISCHEM ABFALL
CHAMBRE, SYSTÈME ET PROCÉDÉ POUR LE TRAITEMENT DE DÉCHETS MÉDICAUX

(30) Priority: 30.12.2020 SE 2051574
(43) Date of publication of application: 06.09.2023
(73) Proprietor: Näslund, Ingemar, 141 39 Huddinge (SE)
(72) Inventor: Näslund, Ingemar, 141 39 Huddinge (SE)
(74) Representative: Brann AB
(86) International application number: PCT/SE2021/051150
(87) International publication number: WO 2022/146212

(56) References cited:
- CN-A- 111 803 693

## Description

### TECHNICAL FIELD

The present invention relates to waste processing, in particular processing of hazardous medical waste using ozone.

### BACKGROUND

Hazardous medical waste, such as infected hospital waste, is in conventional systems typically transported from hospitals by specialized transport vehicles to incinerators, or alternatively dumped directly in landfills. There also exists various conventional on-site equipment for disposing of hazardous waste directly at the hospital site, to avoid transport of the medical waste from the hospital in an infectious condition.

Such on-site equipment typically operates by heating the hazardous waste in autoclaves, or by using ultrasound to heat the waste. Such treatment is energy intensive with high operating cost and may contribute to odors and CO2 emissions. As an alternative, the treatment can be made with ozone. Ozone treatment is less energy demanding with lower operating cost and does not cause as much odor and CO2 emissions. For the past ten years, there has been made available a technology where hazardous waste is exposed to highly concentrated ozone for a short period of time, e.g., 15 minutes, in repeated small batches of waste. After processing, the material is transferred into a container for later transport. In some of these techniques, the hazardous waste is also pre-treated for volume reduction, e.g., by tearing the material into small pieces in a powerful shredder.

Document US7550111 B2 shows a conventional system for ozone treatment.

However, this conventional solution may present a danger to staff handling the medical waste since high concentration levels of ozone are harmful to humans, e.g., if there is a leakage or any accidental breach of the environment used to disinfect the batch of medical waste. A further drawback is that only a relatively small batch of waste can be processed each time and the procedure needs to be restarted several times to process the medical waste. If medical waste is exposed to ozone only for a short period of time, there is also a risk that some of the medical waste is not exposed to enough ozone and therefore will remain in an infectious condition after ozone processing.

Document CN111 803 693 A1 discloses a waste processing chamber configured to process a predetermined amount of medical waste at each session. The process must be interrupted and re-started before the next batch of medical waste can be processed.

Thus, there is a need for an improved chamber, system, and method for processing medical waste. In particular, solutions that allows medical waste, such as infected material, to be disposed of and disinfected on-site within each individual hospital, without the need for the waste being transported from the hospital in an infectious state.

### OBJECTS OF THE INVENTION

An objective of embodiments of the present invention is to provide a solution which mitigates or solves the drawbacks and problems described above.

### SUMMARY OF THE INVENTION

The above and further objectives are achieved by the subject matter of independent claims 1 and 15. Further embodiments are recited in the dependent claims.

At least one advantage of the invention as defined in independent claims 1 and 15 is at least that a better workflow can be achieved, as waste can continuously be fed into the processing chamber. A further advantage is that the risk for personnel involved in the management of medical waste can be reduced, as relatively low concentration of ozone is used. A further advantage is lower requirements for electricity distribution, electricity capacity and lower energy consumption is achieved. Processed material can be stored safely in the hospital for several days, without cold storage, for further transport at a suitable time. There is also an option to check the sterilization level for the whole batch, which is not possible with today's technical solutions.

Claim 9 recites a particular embodiment of claim 1.

Further applications and advantages of embodiments of the invention will be apparent from the following detailed description.

### BRIEF DESCRIPTION OF THE DRAWINGS

**Fig. 1** shows a waste processing chamber 100 according to the one or more embodiments of the disclosure.
**Fig. 2** shows a waste processing system according to one or more embodiments of the present disclosure.
**Fig. 3** illustrates a method according to one or more embodiments of the present disclosure.
**Fig. 4** shows a system with reduced height according to one or more embodiments of the present disclosure.
**Fig. 5A-D** Illustrates movement between positions of a second feeder piston.
**Fig. 6A-B** shows different views of a transporting arrangement according to one or more embodiments of the present disclosure.
**Fig. 7** shows a shredder assembly according to one or more embodiments of the present disclosure.
**Fig. 8** shows an evacuation assembly according to one or more embodiments of the present disclosure.
**Fig. 9A** shows a side view of the evacuation assembly according to one or more embodiments of the present disclosure.
**Fig. 9B** shows a front view of the evacuation assembly according to one or more embodiments of the present disclosure.
**Fig. 10A** shows a shredder assembly 1000 before the waste processing system operates in the loading mode according to one or more embodiments of the present disclosure.
**Fig. 10B** shows a shredder assembly 1000 before or during when the waste processing system operates in the loading mode according to one or more embodiments of the present disclosure.
**Fig. 11** shows an example of when the shredder assembly 1000 receives whole medical waste before the waste processing system operates in the loading mode according to one or more embodiments of the present disclosure.

A more complete understanding of embodiments of the invention will be afforded to those skilled in the art, as well as a realization of additional advantages thereof, by a consideration of the following detailed description of one or more embodiments. It should be appreciated that like reference numerals are used to identify like elements illustrated in one or more of the figures.

### DETAILED DESCRIPTION

An "or" in this description and the corresponding claims is to be understood as a mathematical OR which covers "and" and "or", and is not to be understand as an XOR (exclusive OR). The indefinite article "a" in this disclosure and claims is not limited to "one" and can also be understood as "one or more", i.e., plural.

In the present disclosure the term "processing chamber" denotes a receptacle configured to hold medical waste in an environment. The processing chamber may be implemented in any suitable manner, e.g., as a cylindrical tank or as a room/space. The processing chamber may be made from suitable ozone resistant/compatible materials, such as concrete, stainless steel, Butyl, Chemraz, cross-linked polyethylene (PEX), Ethylene-propylene, Floursilicone, Glass or polycarbonate.

In the present disclosure the term "loading mode" denotes a configuration of the processing chamber such that particles of medical waste can be received via an inlet.

In the present disclosure the term "processing mode" denotes a configuration of the processing chamber where the particles of medical waste are continuously stirred or tumbled around and exposed to ozone at a target ozone concentration level.

In the present disclosure the term "unloading mode" denotes a configuration of the processing chamber where the processed particles of medical waste are moved to an outlet and extracted via the outlet from the processing chamber.

In the present disclosure the terms "ozone generator" or "generator configured to generate ozone" denotes an apparatus or arrangement capable of converting oxygen to ozone. An example of an ozone generator is ultraviolet, UV, light sources, such as a 185 nm UV light such as germicidal lamps available from LightTech LightSources available at https://www.lightsources.com/blog/185nm-uv-lamp-for-ozone-disinfection/. A further example of generators can be found at https://www.ozonetech.com/products generatoes/ict-series. A further example is ozone generators operating on the principle of corona discharging.

### Brief overview of the disclosure

As mentioned in the background section, handling hazardous or infectious waste is cumbersome, costly and may present danger to staff handling the medical waste.

Some conventional systems apply high concentration levels of ozone to relatively small batches of medical waste. The ozone is consumed when the waste is processed, and the concentration level of ozone drops over time and the risk that all particles will not be fully exposed du to compression and clumping of the particles. Another drawback of such solutions is that such high concentration levels of ozone are harmful to humans, e.g., if there is a leakage or any accidental breach of the environment used to disinfect the batch of medical waste. A further drawback is that only a relatively small batch of waste can be processed each time, and the procedure needs to be restarted several times to process the medical waste. I.e. the process needs attention from a user and cannot be left to run autonomously overnight.

The present disclosure improves processing of medical waste by providing a processing chamber configured to operate in any one of a loading mode, a processing mode, and an unloading mode. A constant and relatively low concentration level of ozone is maintained in an environment comprised by the processing chamber to disinfect particles of medical waste using ozone. Further, the particles of medical waste are stirred or tumbled to ensure that all particles are exposed to ozone. Thus, eliminating the risk that all particles would not be fully exposed to ozone due to compression and clumping/aggregation of the particles.

A whole day's production of medical waste with great variation in moisture, bacterial content, virus content, other pathogens and material compositions can be emptied at irregular rates and shredded into particles. A relatively large entire batch can be added over time in a processing chamber and can be exposed to a relatively low concentration of ozone, compared to existing equipment, for a relatively long period of time, such as an hour or several hours if needed. During this process, all particles are separated by mechanical agitation so that all particles are exposed to the target ozone concentration level. This is achieved by providing an ozone generator, that replaces ozone consumed by the process. Further, a particle transporting arrangement is provided for the dual purpose of stirring particles of medical waste in the processing mode and for transporting the particles of medical waste in the unloading mode.

There is a link between ozone concentration and exposure time to achieve disinfection to a desired level. The present disclosure sets an ozone concentration level and find out the required exposure time or vice versa set a time (e.g., 1 - 10 hours) and find out what ozone concentration level is needed for all particles to be disinfected.

Further the processing chamber may comprise a controllable gas inlet configured to provide fresh air and/or oxygen to the processing chamber that can be used to generate ozone and/or ozone generated by a generator arranged outside of the processing chamber. The controllable gas inlet may comprise a separate inlet for air and/or a separate inlet for oxygen and/or a separate inlet for ozone. Additionally, or alternatively, the controllable gas inlet may comprise a combined gas inlet configured to provide any combination of any of air and/or oxygen and/or ozone.

Further the processing chamber may comprise a controllable gas outlet configured to let out gas from the environment in the processing chamber, e.g., to create negative pressure compared to the air pressure outside of the processing chamber, thus reducing the risk of ozone leaking out of the processing chamber.

### Details of the disclosure

Further details of the disclosure are provided by the drawings and the following sections.

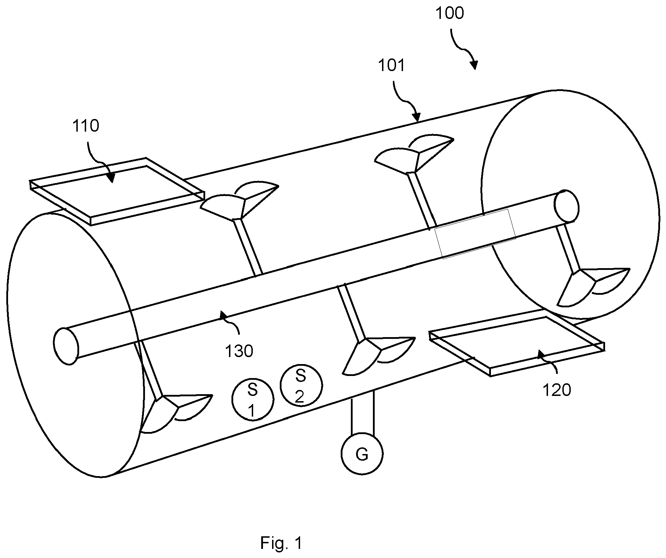

Fig. 1 shows a waste processing chamber 100 according to the one or more embodiments of the disclosure. The waste processing chamber 100 is configured to operate in any one of a loading mode, a processing mode, and an unloading mode.

The processing chamber 100 comprises an inlet 110, an outlet 120 and a particle transporting arrangement 130. The inlet 110 is configured to move between an open position where particles of medical waste can flow into the processing chamber 100 and a closed position where a gastight or hermetic seal is formed between the interior and exterior of the processing chamber 100. The outlet 120 is configured to move between an open position where particles of medical waste can flow out of the processing chamber 100 and a closed position where a gastight or hermetic seal is formed between an environment inside and an environment outside of the processing chamber (100), i.e., the interior and exterior of the processing chamber 100.

In the loading mode the processing chamber is configured such that particles of medical waste can be received via an inlet 110. In other words, in the loading mode the processing chamber is configured with the inlet 110 in the open position where particles of medical waste can can flow into the processing chamber 100.

In the processing mode the processing chamber is configured such that the particles of medical waste are continuously stirred or tumbled by a particle transporting arrangement 130 and exposed to ozone at a target ozone concentration level. In other words, in the processing mode the processing chamber is configured with the inlet 110 in the closed position effectively forming an gastight or hermetic seal. The processing chamber is further configured with an activated/rotating transporting arrangement 130 configured to stir the particles of medical waste inside the processing chamber 100.

In the unloading mode the processing chamber is configured such that the processed particles of medical waste are moved to an outlet 120 by the particle transporting arrangement 130 and extracted via the outlet from the processing chamber. In other words, in the unloading mode the processing chamber is configured with the inlet 110 in the closed position where particles of medical waste can can flow out of the processing chamber 100. The processing chamber is further configured with an activated/rotating transporting arrangement 130 configured to move the particles of medical waste to the outlet 120 for extraction from the processing chamber 100.

The processing chamber 100 typically comprises a receptacle or container 101 configured to hold medical waste in an environment during the processing mode to disinfect particles of medical waste using ozone.

In one embodiment, the receptacle or container 101 is configured to hold medical waste in a sealed or hermetically sealed environment.

The processing chamber 100 may have an elongated shape having a longitudinal axis. The processing chamber may be implemented in any suitable manner, e.g., as a cylindrical tank or as a rectangular room/space, e.g., and may e.g., be made from metal or concrete. Any other suitable material may be used.

The processing chamber 100 comprises the inlet 110 configured to, in the loading mode, to receive the particles of medical waste, and configured to, and in the processing mode to provide a hermetic seal between the environment inside and an environment outside of the processing chamber (100).

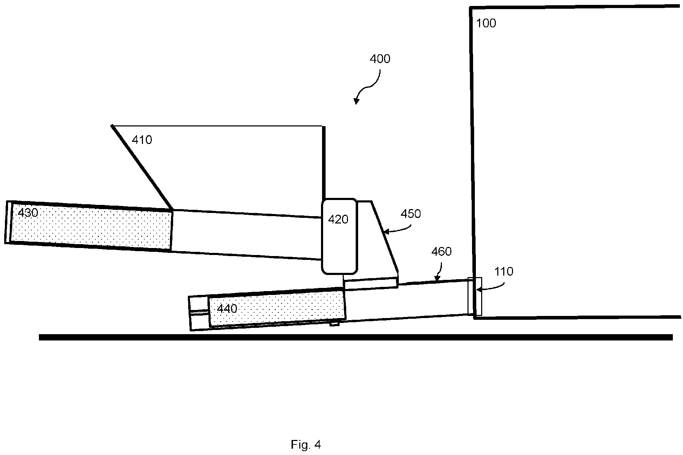

The inlet 110 may be placed at any suitable location on the processing chamber 100, e.g., on a top half of the processing chamber 100 as shown in Fig. 1 or at a lower half of the processing chamber 100 as shown in Fig. 4.

Advantages of placing the inlet 110 on the top half of the processing chamber 100 is that the force of gravity will feed the processed particles of medical waste to the inlet 110 of the waste processing chamber 100, and no separate feeder unit to feed the processed particles of medical waste to the inlet 110 of the waste processing chamber 100 is needed.

Advantages of placing the inlet 110 on the lower half of the processing chamber 100 is that the total height of a waste processing system including the waste processing chamber can be reduced.

The processing chamber 100 further comprises the outlet 120 configured to, in the unloading mode, to extract the particles of medical waste from the processing chamber 100, and configured to, in the loading mode and processing mode, to provide a hermetic seal between the environment inside and the environment outside of the processing chamber 100.

The processing chamber 100 further comprises the particle transporting arrangement 130 configured, in the loading mode and in the processing mode, to stir the particles of medical waste, and further configured, in the unloading mode, to move the particles of medical waste to the outlet 120 for extraction.

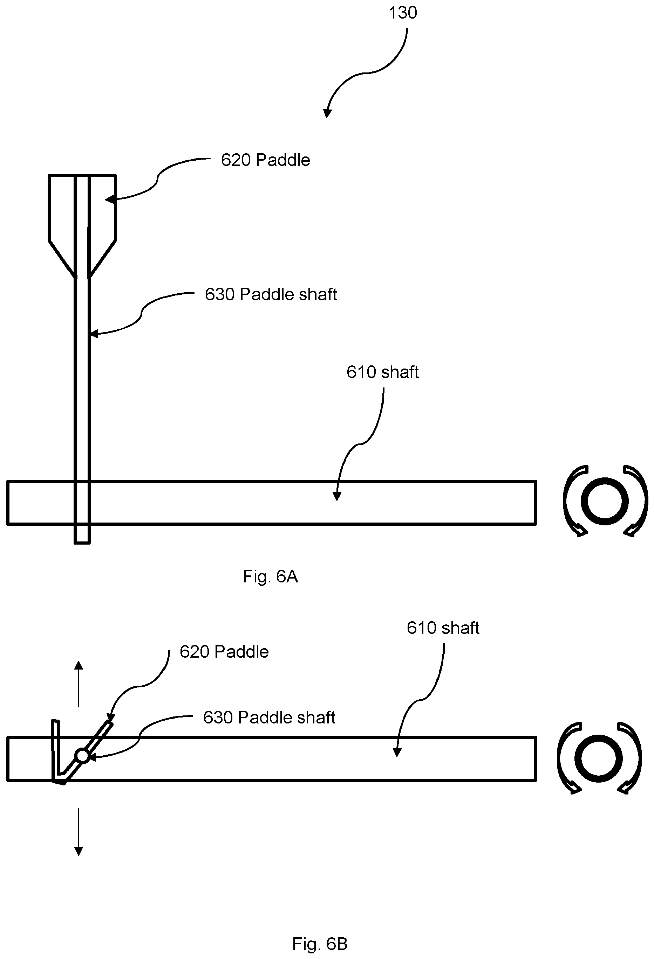

In one embodiment, the transporting arrangement 130 is provided with a shaft 610, having a longitudinal axis parallel to a longitudinal axis of the elongated processing chamber 100, and paddles 620 extending in orthogonal directions to the longitudinal axis of the shaft. The paddles 620 may be mounted on a paddle shaft 630, as shown in Fig. 6A-B, and may be mounted at a fixed angle or controlled to any target angle. In one example the paddles have a flat dovetail shape and may be oriented with an angle between a normal of its flat surface and the longitudinal axis of the elongated processing chamber 100 of ninety degrees in the processing mode. In one further example the paddles have a flat dovetail shape and may be oriented with an angle between a normal of its flat surface and the longitudinal axis of the elongated processing chamber 100 of forty-five degrees in the unloading mode (in the direction towards the outlet). In this manner, the particles of medical waste are stirred in the unloading mode, and moved towards the outlet in the unloading mode.

The processing chamber 100 further comprises or is fluidly coupled to a generator G configured to generate ozone and to maintain a target concentration level of ozone of the environment inside the processing chamber 100. The generator G may be arranged inside the processing chamber 100 or be arranged outside of the processing chamber 100 and fluidly coupled to the inside of the processing chamber 100, thus enabling transfer of generated ozone to the environment inside the processing chamber 100.

In one embodiment, the generator G is releasably attached to the processing chamber (100).

In one embodiment, the processing chamber 100 further comprises one or more sensors S1, S2 configured to measure characteristics of the environment inside of the processing chamber 100. In one embodiment, the one or more sensors S1, S2 comprise at least an ozone sensor configured to measure an ozone concentration level of the environment inside the processing chamber 100. Any suitable number of sensors may be used.

In one embodiment, the one or more sensors S1, S2 comprise at least a temperature sensor configured to measure a temperature of the environment inside the processing chamber 100. In this embodiment, the processing chamber may further comprise a controllable climate control unit (not shown) configured to maintain a target temperature of the environment inside the processing chamber 100. Example interval of target temperatures are 10-30 degrees Celsius.

In one embodiment, the one or more sensors S1, S2 comprise at least a humidity sensor configured to measure a humidity of the environment inside the processing chamber 100. In this embodiment, the processing chamber 100 may further comprise a controllable humidifier configured to maintain a target humidity of the environment inside the processing chamber 100. Example interval of target humidity is 30-100% humidity.

In one embodiment, the processing chamber 100 further comprises inspection window/s. The inspection window/s may in some embodiments be arranged on an upper half of the processing chamber 100. An upper/top half of the chamber 100 may be defined by the direction of gravity and a horizontal plane comprising the longitudinal axis of the processing chamber 100 and being orthogonal to the direction of gravity. The plane splits the processing chamber 100 into an upper half furthest away from center of gravity of Earth, and a lower half relatively closer to the center of gravity of Earth.

In one embodiment, the processing chamber 100 further comprises a controllable gas inlet configured to provide fresh air and/or oxygen and/or to provide ozone to the processing chamber 100. The controllable gas inlet may comprise a separate inlet for air and/or a separate inlet for oxygen and/or a separate inlet for ozone. Additionally, or alternatively, the controllable gas inlet may comprise a combined gas inlet configured to provide any combination of any of air and/or oxygen and/or ozone. Additionally, or alternatively, the processing chamber 100 further comprises a controllable gas outlet configured to let out gas from the environment in the processing chamber 100 and/or to create a negative pressure compared to the atmosphere outside of the processing chamber 100.

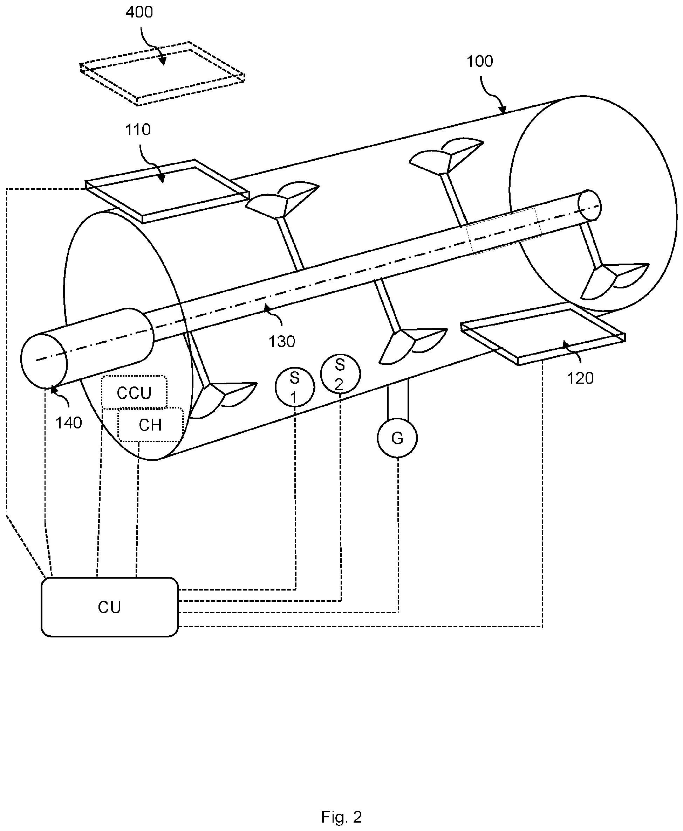

**Fig. 2** shows a waste processing system according to one or more embodiments of the present disclosure. The waste processing system is configured to disinfect particles of medical waste using ozone. The system comprises the waste processing chamber 100, as described in relation to Fig. 1. The system optionally comprises a shredder assembly 400 configured to receive medical waste and process them to particles of medical waste, and to feed the particles of medical waste to the inlet 110 of the waste processing chamber 100. The system further comprises a control unit CU configured to control the processing chamber 100 to operate in any one of a loading mode, a processing mode, and an unloading mode, wherein the control unit CU operating in the processing mode is configured to control the generator G using input received from at least one of the one or more sensors S2, S2. The input may e.g., be indicative of a measured ozone concentration level of the environment inside the processing chamber 100 and/or a measured temperature of the environment inside the processing chamber and/or measured humidity of the environment inside the processing chamber.

In one embodiment, the system further comprises a shredder 420, 720, 1020 configured to receive medical waste and process to particles of medical waste, and to feed the particles of medical waste to the inlet 110 of the waste processing chamber 100.

In one embodiment, the one or more sensors S2, S2 of the processing chamber 100 comprise at least one temperature sensor (not shown) and the system further comprises a controllable climate control unit CCU, wherein the control unit CU, operating in the processing mode, is configured to control the controllable climate control unit using input received from the temperature sensor to maintain a target temperature of the environment inside the processing chamber 100. The target temperature may be stored in a memory of the control unit CU and/or received from input made by a user of the system and/or received by a node via a communications network, e.g., a node in the form of a smartphone.

In one embodiment, the one or more sensors S2, S2 of the processing chamber 100 comprise at least one humidity sensor (not shown) and the system further comprises a controllable humidifier CH, wherein the control unit CU, operating in the processing mode, is configured to control the controllable humidifier using input received from the humidity sensor to maintain a target humidity of the environment inside the processing chamber 100. The target humidity may be stored in a memory of the control unit CU and/or received from input made by a user of the system and/or received by a node via a communications network, e.g., a node in the form of a smartphone.

In one embodiment, the transporting arrangement 130 is provided with a drive unit 140 configured to rotate a shaft of the transporting arrangement 130 in clockwise or anticlockwise direction around the longitudinal axis of the shaft. The drive unit 140 is communicatively coupled to the control unit CU and configured to control rotation of the shaft in response to control signals received from the control unit CU and to provide status of the drive unit 140 and/or transporting arrangement 130, such as revolutions per minute and direction of rotation, to the control unit CU. The drive unit 140 may e.g., be implemented as a servo motor.

In one embodiment, the control unit CU is communicatively coupled to/via a communications network and further configured to transmit status of the system to one or more nodes via the communications network and/or receive commands from the one or more nodes via the communications network. Examples of status is if the process is running or not, measured temperature, humidity, or measured ozone concentration level. Example of commands are commands to start or stop the process.

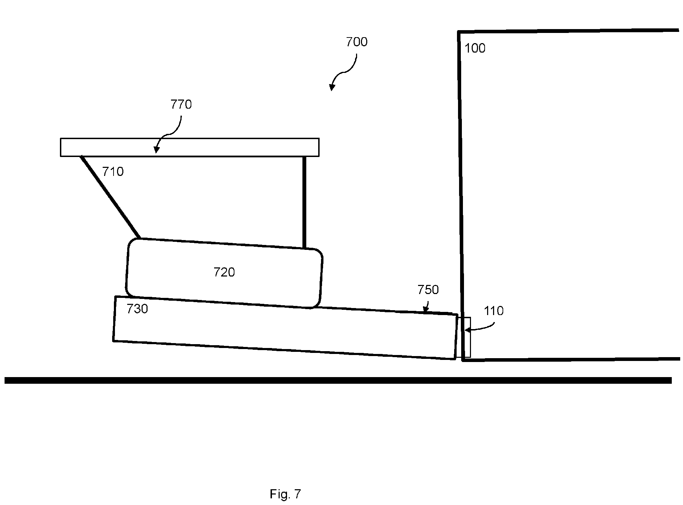

In some implementations of the system, the total height of the room where the system is located is limited. In one embodiment, the inlet 110 is arranged on a lower half of the processing chamber 100. An example of this embodiment is further described in relation to Figs 4 and 7.

The system may comprise further sensors outside of the processing chamber 100 that can detect any ozone leaks, and signal an alarm to the user of the system.

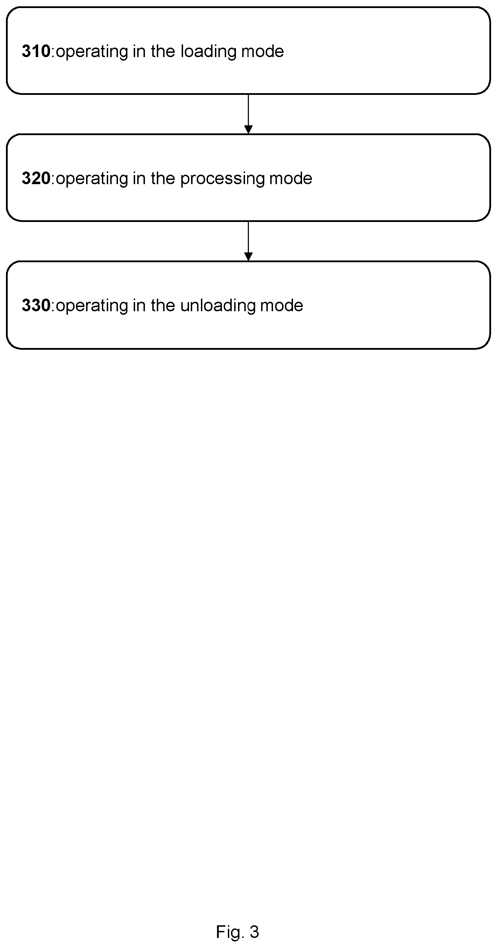

Fig. 3 illustrates a method according to one or more embodiments of the present disclosure. The method is performed by a control unit CU of a system comprising waste processing chamber 100 as described in relation to Fig.1. The waste processing chamber 100 being configured to operate in any one of a loading mode, a processing mode and an unloading mode, the method comprising:
Step 310: operating in the loading mode by controlling the system:
opening or control opening of an inlet 110, in the loading mode, to receive the particles of medical waste, closing or control closing of an outlet 120, in the loading mode, to provide a hermetic seal between an environment inside and an environment outside of the processing chamber (100), and optionally stirring or control stirring of the received particles of medical waste. The inlet may be opened or controlled to open by sending a control signal from the control unit CU to an actuator operating on and mechanically connected to the inlet 110, e.g., a servo motor moving a hatch covering the inlet 110 via a suitable mechanism. The outlet 120 may be closed or controlled to close by sending a control signal from the control unit CU to an actuator operating on and mechanically connected to the outlet, e.g., a servo motor moving a hatch covering the outlet via a suitable mechanism. Particles may be stirred or controlled to stir by sending a control signal from the control unit CU to an actuator operating on and mechanically connected to the particle transporting arrangement 130, e.g., a servo motor 140 rotating a shaft of the particle transporting arrangement 130.

**Step 320:** operating in the processing mode by controlling the system:
closing or control closing of the inlet 110 to provide a hermetic seal between an environment inside and an environment outside of the processing chamber 100, stirring the received particles of medical waste, and generating ozone and maintaining a target concentration level of ozone of the environment inside the processing chamber 100, e.g. during a set time of 1-10 hours.

The inlet 110 may be closed or controlled to close by sending a control signal from the control unit CU to the actuator operating on and mechanically connected to the inlet 110, e.g., a servo motor moving a hatch covering the outlet via a suitable mechanism. Particles may be stirred or controlled to be stirred by sending a control signal from the control unit CU to the actuator operating on and mechanically connected to the particle transporting arrangement 130, e.g., a servo motor 140 rotating a shaft of the particle transporting arrangement 130. Ozone may be generated and maintained or controlled to be generated and maintained at a target concentration level of ozone, by receiving control signals from the one or more sensors S1, S2, the signals being indicative of a measured concentration level of ozone of the environment inside the processing chamber 100. Further, the measured and received concentration level is compared to a target concentration level, e.g., retrieved from a memory of the control unit CU. Further, a control signal indicative of a desired generation of ozone, resulting in the target level of ozone being achieved, is sent to the generator G. In one example, if the comparison shows that measured concentration level of ozone is below the target concentration level, a control signal indicative of a relatively higher rate of ozone generation is sent to the generator G. In one further example, if the comparison shows that measured concentration level of ozone is above the target concentration level, a control signal indicative of a relatively lower rate of ozone generation is sent to the generator G.

**Step 330:** operating in the unloading mode by controlling the system:
opening or controlling the opening of the outlet 120 to extract the particles of medical waste from the processing chamber 100, and stirring or controlling the stirring of the particles of medical waste and to move them towards the outlet 120.

The outlet 120 may be opened or be controlled to open by sending a control signal from the control unit CU to an actuator operating on and mechanically connected to the outlet 120, e.g., a servo motor moving a hatch covering the outlet 120 via a suitable mechanism. The particles may be stirred and moved or be controlled to be stirred and moved towards the outlet 120 by sending a control signal from the control unit CU to the actuator operating on and mechanically connected to the particle transporting arrangement 130, e.g., a servo motor 140 rotating a shaft of the particle transporting arrangement 130. Paddles may be mounted on a paddle shaft, as shown in Fig. 6A-B, and may be controlled to be oriented with an angle between a normal of its flat surface and the longitudinal axis of the elongated processing chamber 100 of e.g., forty-five degrees in the unloading mode (in the direction towards the outlet). In this manner, the particles of medical waste are stirred and moved towards the outlet.

In one embodiment, generating the ozone and maintaining a target concentration level of ozone or controlling generation of the ozone and maintenance the target concentration level of ozone is performed using the measured ozone concentration level.

In one example, the measured ozone concentration level indicates a level below a target level, and the rate of ozone generation is increased by the generator G. In one further example, the measured ozone concentration level indicates a level above a target level, and the rate of ozone generation is decreased by the generator G. In other words, the measured ozone concentration level is used to maintain a constant ozone concentration level by the generator G.

The system may comprise further sensors outside of the processing chamber 100 that can detect any ozone leaks, and signal an alarm to the user of the system.

In one embodiment, the one or more sensors S1, S2 comprise at least a temperature sensor configured to measure a temperature of the environment inside the processing chamber 100, wherein the system further comprises a controllable climate control unit configured to maintain a target temperature of the environment inside the processing chamber 100, wherein the method further comprises maintaining the target temperature using the measured temperature.

Maintaining the target temperature using the measured temperature may be performed by receiving control signals, by the CU, from the one or more sensors S1, S2, the signals being indicative of a measured temperature of the environment inside the processing chamber 100. Further, the measured temperature is compared, by the CU, to a target temperature, e.g., retrieved from a memory of the control unit CU. Further, a control signal indicative of a desired climate control, resulting in the target temperature being achieved, is sent to the climate control unit CCU. In one example, if the comparison shows that measured temperature is below the target temperature, a control signal indicative of generating heat is sent to the climate control unit CCU. In one further example, if the comparison shows that measured temperature is above the target temperature level, a control signal indicative of cooling is sent to the climate control unit CCU.

In one embodiment, the one or more sensors S1, S2 comprise at least a humidity sensor configured to measure humidity of the environment inside the processing chamber 100, wherein the system further comprises a controllable humidifier configured to maintain a target humidity of the environment inside the processing chamber (100), wherein the method further comprises maintaining the target humidity using the measured humidity.

Maintaining the target humidity using the measured humidity may be performed by receiving control signals from the one or more sensors S1, S2, the signals being indicative of a measured humidity of the environment inside the processing chamber 100. Further, the measured humidity is compared to a target humidity, e.g., retrieved from a memory of the control unit CU. Further, a control signal indicative of a desired humidity control, resulting in the target humidity being achieved, is sent to the humidity control unit. In one example, if the comparison shows that measured humidity is below the target humidity, a control signal indicative of generating humidity is sent to the climate control unit CCU. In one further example, if the comparison shows that measured temperature is above the target temperature level, a control signal indicative of reducing humidity is sent to the climate control unit CCU.

In one embodiment, and before initiating operation in the unloading mode, the method further comprises opening a controllable gas inlet configured to provide fresh air to the processing chamber 100 and/or opening a controllable gas outlet configured to let out gas from the environment in the processing chamber 100. The controllable gas inlet may be opened by sending a control signal from the control unit CU to an actuator operating on and mechanically connected to the controllable gas inlet, e.g., a servo motor moving a valve via a suitable mechanism. The controllable gas outlet may be opened by sending a control signal from the control unit CU to an actuator operating on and mechanically connected to the controllable gas outlet, e.g., a servo motor moving a valve.

**Fig. 4** shows a system with reduced height according to one or more embodiments. In embodiments where the system comprises a shredder 420 configured to receive medical waste and process to particles of medical waste, and to feed the particles of medical waste to the inlet 110 of the waste processing chamber 100. The total heigh of the system may be as much as 3.5 meters. It may then be desirable to find a configuration that requires less ceiling height.

In this configuration, the inlet 110 is located on the lower half of the processing chamber 100, e.g., at the bottom of the processing chamber 100. A shredder assembly 400 is coupled to the inlet 110 of the processing chamber 100. The shredder assembly 400 comprises a hopper 410, a shredder/shredder unit 420.

In one embodiment the shredder assembly 400 further comprises and one or more feeder units, e.g., a first feeder piston or a first transport screw 430 and/or a second feeder piston or a second transport screw 440.

Optionally, the shredder assembly 400 further comprises a first inspection window 450 and/or a second inspection window 460.

The hopper 410 is configured to receive whole medical waste.

In one example, a first feeder unit, e.g., the first feeder piston or a first transport screw 430, is configured to feed the received whole medical waste to the shredder unit 420. Additionally or alternatively, a second feeder unit, e.g., the second feeder piston or second transport screw 440 is configured to feed the particles of medical waste to the inlet 110 of the coupled waste processing chamber 100.

In one further example, first feeder piston 430 is configured to feed the received whole medical waste to the shredder unit 420. The shredder unit 420 is configured to process the whole medical waste to particles of medical waste. The second feeder piston 440 is configured to feed the particles of medical waste to the inlet 110 of the coupled waste processing chamber 100.

Optionally, the shredder assembly 400 is provided with a first inspection window 450 where shredded particles of medical waste directly after the shredder unit 420 can be inspected. Optionally, the shredder assembly 400 is further provided with a second inspection window 460 where shredded particles of medical waste fed to the inlet 110 can be inspected.

In one embodiment, the first feeder piston 430 and/or the second feeder piston 440 is tilted relative to a horizontal plane, to allow fluid to escape via drainage holes.

In the embodiment shown in Fig. 4, the first and second feeder units 430, 440 are tilted relative to the horizontal plane, but it is understood that the first and second feeder units 430, 440 may equally be level with the horizontal plane and/or tilted the opposite way to what is shown in Fig. 4.

In one embodiment, the first feeder piston 430 is controlled, by the control unit CU, to move with a force adapted continuously to a certain power resistance retrieved from the CU and relatively slower than what the second feeder piston 440 is controlled, by the control unit CU, to move.

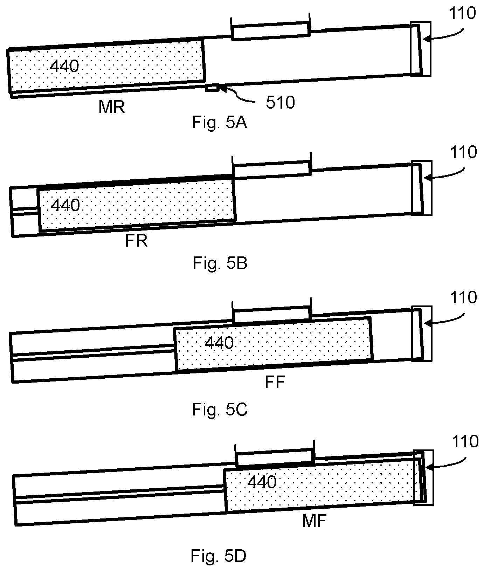

Fig. 5A-D Illustrates movement between positions of the second feeder piston 440.

In one embodiment, the second feeder piston 440 is arranged to move to positions between a maximum retracted position MR illustrated in Fig. 5A, a feeding retracted position FR illustrated in Fig. 5B, a feeding forward position FF illustrated in Fig. 5C and a maximum forward position MF illustrated in Fig 5D.

When the processing chamber is operating in the loading mode, the second feeder piston 440 is controlled, by the control unit CU, to move between the feeding retracted position FR and the feeding forward position FF.

When the processing chamber is operating in the processing mode, the second feeder piston 440 is controlled, by the control unit CU, to move to the maximum forward position MF, thereby hermetically sealing off the inlet 110. In other words, creating a hermetic seal between the atmosphere inside and outside the processing chamber 100.

When the processing chamber is operating in the unloading mode, the second feeder piston 440 is controlled, by the control unit CU, to move to the maximum retracted position MR, thereby allowing the inspection windows 450 and 460 can be opened and the entire shredder assembly unit 400 to be cleaned with rinsing and the fluid trapped in the second feeder piston housing to drain through a drain 510.

**Fig. 6A** shows a side view of a transporting arrangement 130 according to one or more embodiments of the present disclosure. In this embodiment, the transporting arrangement 130 is provided with a shaft or drive shaft 610, having a longitudinal axis parallel to a longitudinal axis of the elongated processing chamber 100, and paddle/s 620 extending in orthogonal directions to the longitudinal axis of the shaft. Each of the paddle/s 620 may be mounted on a paddle shaft 630. The shaft or drive shaft 610 may be rotated around its longitudinal axis by a drive unit 140, e.g., a servo motor.

**Fig. 6B** shows a top view of a transporting arrangement 130 according to one or more embodiments of the present disclosure. As can be seen from Fig. 6B, the paddle/s 620 are formed with a V-shape. The paddle/s 620 are formed with one leg of the V-shape perpendicular to the longitudinal axis of the shaft or drive shaft 610 and the second leg at an angle relative to the one leg, e.g., at an angle of 30-60 degrees.

This has the effect that when the shaft or drive shaft 610 rotates in one direction, illustrated by the upward facing arrow in Fig. 6B, the particles of medical waste are stirred in the processing mode. When the shaft or drive shaft 610 rotates in the opposite direction, illustrated by the downward facing arrow in Fig. 6B, the particles of medical waste the particles of medical waste are transported, e.g., in the unloading mode.

It is understood that the shaft or drive shaft 610 may be provided with any suitable number of paddle/s 620 and/or paddle shafts 630 without deviating from the scope of the invention.

**Fig. 7** shows a shredder assembly 700 according to one or more embodiments of the present disclosure.

A shredder assembly 700 is coupled to the inlet 110 of the processing chamber 100, which is configured to receive medical waste and process to particles of medical waste, and to feed the processed particles of medical waste to the inlet 110 of the waste processing chamber 100. The shredder assembly 400 comprises a hopper 710 and a shredder unit 720 configured to receive medical waste and process to particles of medical waste, similarly to what is shown in Fig. 4. In one embodiment. the shredder assembly 700 further comprises at least one feeder unit 730 to feed the processed particles of medical waste to the inlet 110 of the waste processing chamber 100. The feeder unit 730 may e.g., be a screw conveyor or piston/s as shown in Fig. 4. The feeder unit 730 may be provided with a low-friction coating such as polymer coating, to allow the particles of medical waste to move into the processing chamber 100 more easily.

In this configuration, the inlet 110 is located on the lower half of the processing chamber 100, e.g., at the bottom of the processing chamber 100. Optionally, the shredder assembly 700 further comprises a first inspection window 750.

The inlet 110 may be placed at any suitable location on the processing chamber 100, e.g., on a top half of the processing chamber 100 as shown in Fig. 1 or at a lower half of the processing chamber 100 as shown in Fig. 4.

Advantages of placing the inlet 110 on the top half of the processing chamber 100 is that the force of gravity will feed the processed particles of medical waste to the inlet 110 of the waste processing chamber 100, and no separate feeder unit to feed the processed particles of medical waste to the inlet 110 of the waste processing chamber 100 is needed.

Advantages of placing the inlet 110 on the lower half of the processing chamber 100 is that the total height of a waste processing system including the waste processing chamber can be reduced.

The hopper 710 is configured to receive whole medical waste. The shredder unit 720 is configured to process the whole medical waste to particles of medical waste. The feeder unit 730 is configured to feed the particles of medical waste to the inlet 110 of the coupled waste processing chamber 100.

Optionally, the shredder assembly 700 is provided with a lid 770 providing a hermetic seal between the interior of the hopper and the surrounding atmosphere. The hopper 710, the shredder 720, the feeder unit 730 and the lid 770 then forms a hermetically sealed enclosure. In this embodiment, the method of Fig. 3 in **Step 320** may further comprise operating in the processing mode by controlling the system to open or control opening of the inlet 110 to allow the target concentration level of ozone of the environment inside the processing chamber 100 to enter the shredder assembly 700. This sterilizes the inside of the shredder assembly 700 and/or removes any unwanted odor.

Optionally, the shredder assembly 700 is provided with a first inspection window 750 where shredded particles of medical waste directly after the shredder unit 720 can be inspected.

In one embodiment, the feeder unit 730 is tilted relative to a horizontal plane, e.g., the floor shown as a thick black line, to allow fluid to flow in a desired direction.

In one embodiment, the shredder unit 720 and/or the feeder unit 730 is controlled, by the control unit CU.

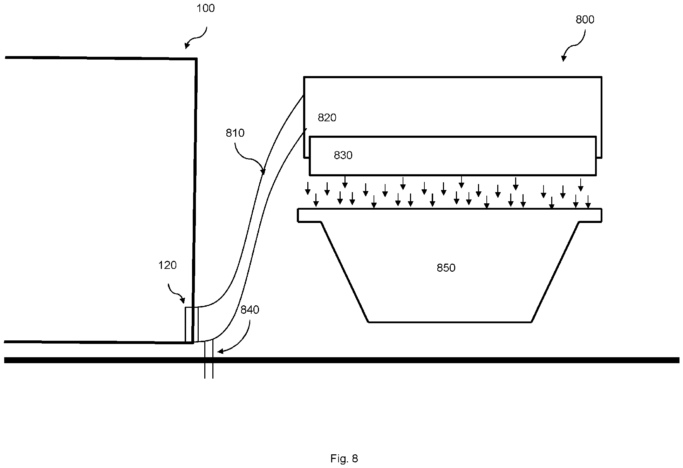

**Fig. 8** shows an evacuation assembly 800 according to one or more embodiments of the present disclosure.

An evacuation assembly 800 is coupled to the outlet 120 of the processing chamber 100, which is configured to extract the processed/treated particles of medical waste from the processing chamber 100, and to move the extracted particles of medical waste to a storage container 850, typically for removal of particles of medical waste from the site of the waste processing chamber 100.

The evacuation assembly 800 typically comprises a pressure tank 820, configured to hold a pressure below atmospheric pressure, i.e., a vacuum. The evacuation assembly 800 typically further comprises coupling means/unit 810, arranged to couple and/or hermetically couple the outlet 120 of the processing chamber 100 to the pressure tank 820 to allow a flow of particles from the processing chamber 100 to the pressure tank 820. The coupling means/unit 810 may e.g., comprise a conduit member such as a tube or pipe. By providing pressure below atmospheric pressure, this allows the evacuation assembly 800 to suck particles of medical waste from the processing chamber 100, thereby extracting the processed/treated particles of medical waste from the processing chamber 100.

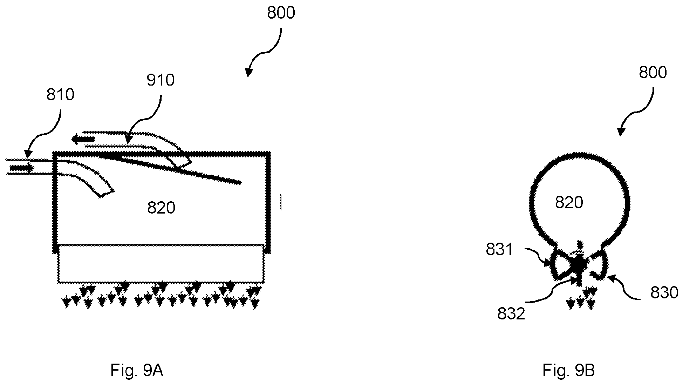

The evacuation assembly 800 further comprises a sluice unit 830 configured_move the extracted particles of medical waste from the pressure tank 820 to the storage container 850, whilst forming a seal to the pressure tank 820 to maintain the vacuum in the pressure tank 820. Details of the sluice unit 830 is further provided in relation to Fig. 9.

The pressure tank 820 and the sluice unit 830 are typically arranged together as an assembly. The pressure tank 820 and the sluice unit 830 may advantageously be placed over the_storage container 850. When the particles of medical waste are moved from the tank, gravity will force the particles of medical waste to fall into the container.

The evacuation assembly 800 may optionally further comprise a drain 840, configured to drain fluid from the coupling means/unit 810. The drain 840 may optionally be provided with a valve that can be placed in an open or closed position, to maintain the vacuum in the coupling means/unit 810 and pressure tank 820.

Fig. 9A shows a side view of the evacuation assembly 800 according to one or more embodiments of the present disclosure. To generate a vacuum in the pressure tank that the coupling means/unit 810 use to extract particles from the processing chamber 100, the evacuation assembly 800 is provided with second coupling means/unit 910. The second coupling means/unit 910 may e.g., comprise a conduit member such as a tube or pipe. The second coupling means/unit 910 generates a vacuum or a pressure below atmospheric pressure by extracting gas comprised by the pressure tank 820.

**Fig. 9B** shows a front view of the evacuation assembly 800 according to one or more embodiments of the present disclosure. The sluice unit 830 is in this embodiment formed as a tube comprising a gate member, shaped similar to a revolving door, and is provided with gas tight seals. The gate member rotates around the central axis of the tube and inside the tube. As the tube is provided with at least a first one opening facing the pressure tank 820 and a second opening facing the storage container 850, the extracted particles of medical waste falls into the sluice unit 830 via the first opening, and to the storage container 850 through the second opening, as the gate member rotates.

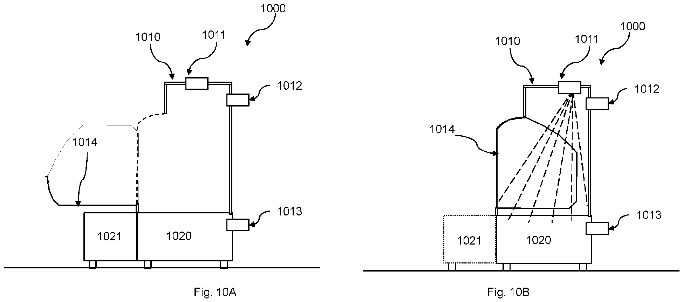

**Fig. 10A** shows a shredder assembly 1000 before the waste processing system operates in the loading mode according to one or more embodiments of the present disclosure. The shredder assembly 1000 corresponds to the shredder assemblies 400, 700 shown in Fig. 4 and 7 respectively, and the features described below may equally be applied to the shredder assemblies 400, 700. The shredder assembly 1000 comprises a shredder 1020 configured to receive medical waste and process to particles of medical waste. The shredder 1020 may comprise a drive unit or be coupled to an external drive unit 1021 via a transmission arrangement, such as a shaft, chain and sprockets or pulleys and belt. The shredder assembly 1000 further comprises a hopper 1010 configured to receive whole medical waste. The hopper 1010 is coupled to the shredder 1020 thereby allowing whole medical waste to reach/be loaded into the shredder 1020 for processing to particles of medical waste. The hopper 1010 may be coupled directly depending on gravitational force to move the waste or be coupled via a feeder unit as described above. The hopper 1010 is in one embodiment provided with a hatch/lid 1014, shown in an open position in Fig. 10A. The hopper 1010 is configured to receive medical waste when the hatch/lid 1014 is positioned in the open position. The hopper 1010 may optionally comprise one or more exhausts 1012, 1013 configured to ventilate gas from the interior of the hopper 1010. The hopper 1010 may further optionally comprise a cleaning unit 1011, e.g., a nozzle coupled to water under pressure. The cleaning unit 1011 is configured to spray the interior of the hopper 1010 and/or the shredder unit 1020 with cleaning fluid, such as water with or without detergent.

**Fig. 10B** shows a shredder assembly 1000 before or during when the waste processing system operates in the loading mode according to one or more embodiments of the present disclosure. The shredder assembly 1000 corresponds to the shredder assemblies 400, 700 shown in Fig. 4 and 7 respectively, and the features described below may equally be applied to the shredder assemblies 400, 700. The shredder assembly 1000 may comprises the same features as described in relation to Fig. 10A. In Fig. 10B, the hatch/lid 1014 is positioned in a closed position. Preferably, the cleaning unit 1011 is configured to spray the interior of the hopper 1010 and/or the shredder unit 1020 with cleaning fluid only when the hatch/lid 1014 is positioned in the closed position

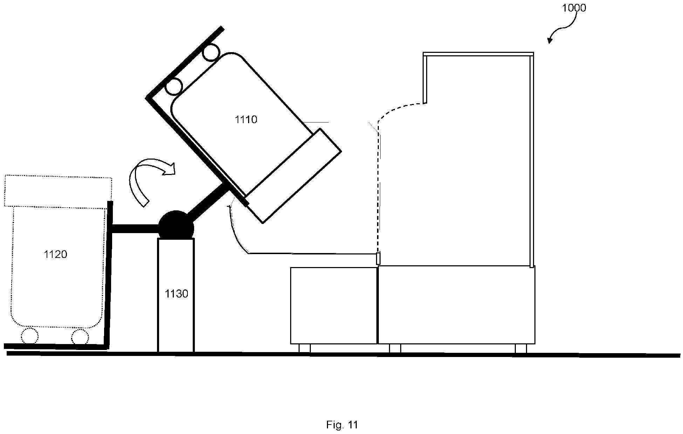

**Fig. 11** shows an example of when the shredder assembly 1000 receives whole medical waste before the waste processing system operates in the loading mode according to one or more embodiments of the present disclosure.

A bin actuator unit 1130 may be configured to empty bins/containers comprising whole medical waste into the hopper 1010 when 1the hatch/lid 1014 is positioned in the open position.

## Claims

1. A waste processing chamber (100) configured to operate in any one of a loading mode, a processing mode and an unloading mode, further configured to provide, in the processing mode, an environment to disinfect particles of medical waste using ozone, the processing chamber comprising:
an inlet (110) configured to, in the loading mode, to receive the particles of medical waste, and configured to, in the processing mode and unloading mode to provide a hermetic seal between the environment inside and an environment outside of the processing chamber (100),
an outlet (120) configured to, in the unloading mode, to extract the particles of medical waste from the processing chamber (100), and configured to, in the loading mode and processing mode, to provide a hermetic seal between the environment inside and the environment outside of the processing chamber (100),
a particle transporting arrangement (130) configured, in the loading mode and in the processing mode, to stir the particles of medical waste, and further configured, in the unloading mode, to move the particles of medical waste to the outlet (120) for extraction,
one or more sensors (S1, S2) configured to measure characteristics of the environment inside the processing chamber (100), wherein the one or more sensors (S1, S2) comprise at least a temperature sensor configured to measure a temperature of the environment inside the processing chamber (100),
wherein the processing chamber (100) is further fluidly coupled to or comprising a generator (G) configured to generate ozone and to maintain a target concentration level of ozone of the environment inside the processing chamber (100), wherein the processing chamber further comprises a controllable climate control unit configured to maintain a target temperature of the environment inside the processing chamber (100).

2. A waste processing chamber according to claim 1, wherein the one or more sensors (S1, S2) comprise at least an ozone sensor configured to measure an ozone concentration level of the environment inside the processing chamber (100).

3. A waste processing chamber according to any of the preceding claims, wherein the generator (G) is releasably attached to the processing chamber (100).

4. A waste processing chamber according to any of the preceding claims, wherein the one or more sensors (S1, S2) comprise at least a humidity sensor configured to measure a humidity of the environment inside the processing chamber (100).

5. A waste processing chamber according to any of the preceding claims, further comprising a controllable humidifier configured to maintain a target humidity of the environment inside the processing chamber (100).

6. A waste processing chamber according to any of the preceding claims, wherein the processing chamber (100) is provided with inspection windows arranged on an upper half of the processing chamber (100.

7. A waste processing chamber according to any of the preceding claims, wherein the transporting arrangement (130) is provided with a shaft, having a longitudinal axis parallel to a longitudinal axis of the processing chamber (100), and paddles extending in orthogonal directions to the longitudinal axis of the shaft.

8. A waste processing chamber according to any of the preceding claims, wherein the processing chamber further comprises:
a controllable gas inlet configured to provide fresh air to the processing chamber (100),
a controllable gas outlet configured to let out gas from the environment in the processing chamber (100).

9. A waste processing system configured to disinfect particles of medical waste using ozone, the system comprising:
the waste processing chamber (100) according to claim 1,
a control unit (CU) configured to control the processing chamber (100) to operate in any one of a loading mode, a processing mode, and an unloading mode, wherein the control unit (CU) operating in the processing mode is configured to control the generator (G) using input received from at least one of the one or more sensors (S2, S2), the input being indicative of a measured ozone concentration level inside the processing chamber (100), wherein the one or more sensors (S2, S2) comprise at least one temperature sensor and the system further comprises a controllable climate control unit, wherein the control unit (CU) operating in the processing mode is configured to control the controllable climate control unit using input received from the temperature sensor to maintain a target temperature of the environment inside the processing chamber (100).

10. The system according to claim 9, wherein the one or more sensors (S2, S2) comprises at least one humidity sensor and the system further comprises a controllable humidifier, wherein the control unit (CU) operating in the processing mode is configured to control the controllable humidifier using input received from the humidity sensor to maintain a target humidity of the environment inside the processing chamber (100).

11. The system according to any of claims 9-10, wherein the control unit (CU) is communicatively coupled to a communications network and further configured to transmit status of the system to one or more nodes via the communications network and/or receive commands from the one or more nodes via the communications network.

12. The system according to any of claims 9-11, wherein the inlet (110) is arranged on a lower half of the processing chamber (100).

13. The system according to any of claims 9-12, further comprising a shredder assembly (700) fluidly coupled to the inlet (110) of the processing chamber (100), which is configured to receive medical waste and process to particles of medical waste, and to feed the processed particles of medical waste to the inlet (110) of the waste processing chamber (100).

14. The system according to any of claims 9-13, further comprising an evacuation assembly (800) fluidly coupled to the outlet (120) of the processing chamber (100), which is configured to extract the processed/treated particles of medical waste from the processing chamber (100), and to move the extracted particles of medical waste to a storage container (850).

15. A method performed by a control unit (CU) of a system comprising a waste processing chamber (100) according to claim 1, the waste processing chamber (100) being configured to operate in any one of a loading mode, a processing mode and an unloading mode, wherein the one or more sensors (S1, S2) comprise at least a temperature sensor configured to measure a temperature of the environment inside the processing chamber (100), wherein the system further comprises a controllable climate control unit configured to maintain a target temperature of the environment inside the processing chamber (100), the method comprising:
operating in the loading mode by:
opening an inlet (110), in the loading mode, to receive the particles of medical waste,
closing an outlet (120), in the loading mode, to provide a hermetic seal between an environment inside and an environment outside of the processing chamber (100),
stirring, in the loading mode, the received particles of medical waste,
operating in the processing mode by:
closing the inlet (110), in the processing mode, to provide a hermetic seal between an environment inside and an environment outside of the processing chamber (100),
stirring, in the processing mode, the received particles of medical waste,
generating, in the processing mode, ozone and maintaining a target concentration level of ozone of the environment inside the processing chamber (100),
operating in the unloading mode by:
opening the outlet (120), in the unloading mode, to extract the particles of medical waste from the processing chamber (100),
wherein the method further comprises maintaining the target temperature using the measured temperature.

16. A method according to claim 15, wherein the one or more sensors (S1, S2) comprise at least an ozone sensor configured to measure an ozone concentration level of the environment inside the processing chamber (100), wherein generating the ozone and maintaining a target concentration level of ozone is performed using the measured ozone concentration level.

17. A method according to any of the claims 15-16, wherein the one or more sensors (S1, S2) comprise at least a humidity sensor configured to measure humidity of the environment inside the processing chamber (100), wherein the system further comprises a controllable humidifier configured to maintain a target humidity of the environment inside the processing chamber (100), wherein the method further comprises maintaining the target humidity using the measured humidity.

18. A method according to any of the preceding claims, wherein:
operating in the unloading mode further comprises:
opening a controllable gas inlet configured to provide fresh air to the processing chamber (100) and opening a controllable gas outlet configured to let out gas from the environment in the processing chamber (100).

## Patentansprüche

1. Abfallverarbeitungskammer (100), die dazu konfiguriert ist, in einem beliebigen eines Belademodus, eines Verarbeitungsmodus und eines Entlademodus betrieben zu werden, ferner dazu konfiguriert ist, in dem Verarbeitungsmodus eine Umgebung zum Desinfizieren von Partikeln medizinischen Abfalls unter Verwendung von Ozon bereitzustellen, wobei die Verarbeitungskammer Folgendes umfasst:
einen Einlass (110), der in dem Belademodus dazu konfiguriert ist, die Partikel medizinischen Abfalls zu erhalten, und in dem Verarbeitungsmodus und
Entlademodus dazu konfiguriert ist, eine hermetische Dichtung zwischen der Umgebung innerhalb und einer Umgebung außerhalb der Verarbeitungskammer (100) bereitzustellen,
einen Auslass (120), der in dem Entlademodus dazu konfiguriert ist, Partikel medizinischen Abfalls aus der Verarbeitungskammer (100) zu extrahieren, und in dem Belademodus und Verarbeitungsmodus dazu konfiguriert ist, eine hermetische Dichtung zwischen der Umgebung innerhalb und der Umgebung außerhalb der Verarbeitungskammer (100) bereitzustellen,
eine Partikeltransportanordnung (130), die in dem Lademodus und in dem Verarbeitungsmodus dazu konfiguriert ist, die Partikel medizinischen Abfalls zu rühren, und ferner in dem Entlademodus dazu konfiguriert ist, die Partikel medizinischen Abfalls zur Extraktion zu dem Auslass (120) zu bewegen,
einen oder mehrere Sensoren (S1, S2), die dazu konfiguriert sind, Eigenschaften der Umgebung innerhalb der Verarbeitungskammer (100) zu messen, wobei der eine oder die mehreren Sensoren (S1, S2) mindestens einen Temperatursensor umfassen, der dazu konfiguriert ist, eine Temperatur der Umgebung innerhalb der Verarbeitungskammer (100) zu messen,
wobei die Verarbeitungskammer (100) ferner fluidisch an einen Generator (G) gekoppelt ist oder einen Generator (G) umfasst, der dazu konfiguriert ist, Ozon zu erzeugen und ein Zielkonzentrationsniveau von Ozon der Umgebung innerhalb der Verarbeitungskammer (100) aufrechtzuerhalten, wobei die Verarbeitungskammer ferner eine steuerbare Klimasteuereinheit umfasst, die dazu konfiguriert ist, eine Zieltemperatur der Umgebung innerhalb der Verarbeitungskammer (100) aufrechtzuerhalten.

2. Abfallverarbeitungskammer nach Anspruch 1, wobei der eine oder die mehreren Sensoren (S1, S2) mindestens einen Ozonsensor umfassen, der dazu konfiguriert ist, ein Ozonkonzentrationsniveau der Umgebung innerhalb der Verarbeitungskammer (100) zu messen.

3. Abfallverarbeitungskammer nach einem der vorhergehenden Ansprüche, wobei der Generator (G) lösbar an der Verarbeitungskammer (100) befestigt ist.

4. Abfallverarbeitungskammer nach einem der vorhergehenden Ansprüche, wobei der eine oder die mehreren Sensoren (S1, S2) mindestens einen Feuchtigkeitssensor umfassen, der dazu konfiguriert ist, eine Feuchtigkeit der Umgebung innerhalb der Verarbeitungskammer (100) zu messen.

5. Abfallverarbeitungskammer nach einem der vorhergehenden Ansprüche, ferner umfassend einen steuerbaren Befeuchter, der dazu konfiguriert ist, eine Zielfeuchtigkeit der Umgebung innerhalb der Verarbeitungskammer (100) aufrechtzuerhalten.

6. Abfallverarbeitungskammer nach einem der vorhergehenden Ansprüche, wobei die Verarbeitungskammer (100) mit Inspektionsfenstern versehen ist, die in einer oberen Hälfte der Verarbeitungskammer (100) angeordnet sind.

7. Abfallverarbeitungskammer nach einem der vorhergehenden Ansprüche, wobei die Transportanordnung (130) mit einer Welle, die eine Längsachse parallel zu einer Längsachse der Verarbeitungskammer (100) aufweist, und Schaufeln, die sich in orthogonale Richtungen zu der Längsachse der Welle erstrecken, versehen ist.

8. Abfallverarbeitungskammer nach einem der vorhergehenden Ansprüche, wobei die Verarbeitungskammer ferner Folgendes umfasst:
einen steuerbaren Gaseinlass, der dazu konfiguriert ist, frische Luft an die Verarbeitungskammer (100) bereitzustellen,
einen steuerbaren Gasauslass, der dazu konfiguriert ist, Gas von der Umgebung in der Verarbeitungskammer (100) abzulassen.

9. Abfallverarbeitungssystem, das dazu konfiguriert ist, Partikel medizinischen Abfalls unter Verwendung von Ozon zu desinfizieren, wobei das System Folgendes umfasst:
die Abfallverarbeitungskammer (100) nach Anspruch 1,
eine Steuereinheit (CU), die dazu konfiguriert ist, die Verarbeitungskammer (100) dazu zu steuern, in einem beliebigen eines Belademodus, eines Verarbeitungsmodus und eines Entlademodus betrieben zu werden, wobei die Steuereinheit (CU), die in dem Verarbeitungsmodus betrieben wird, dazu konfiguriert ist, den Generator (G) unter Verwendung einer Eingabe, die von mindestens einem des einen oder der mehreren Sensoren (S2, S2) erhalten wird, zu steuern, wobei die Eingabe ein gemessenes Ozonkonzentrationsniveau innerhalb der Verarbeitungskammer (100) angibt, wobei der eine oder die mehreren Sensoren (S2, S2) mindestens einen Temperatursensor umfassen und
das System ferner eine steuerbare Klimasteuereinheit umfasst, wobei die Steuereinheit (CU), die in dem Verarbeitungsmodus betrieben wird, dazu konfiguriert ist, die steuerbare Klimasteuereinheit unter Verwendung einer Eingabe, die von dem Temperatursensor erhalten wird, dazu zu steuern, eine Zieltemperatur der Umgebung innerhalb der Verarbeitungskammer (100) aufrechtzuerhalten.

10. System nach Anspruch 9, wobei der eine oder die mehreren Sensoren (S2, S2) mindestens einen Feuchtigkeitssensor umfassen und das System ferner einen steuerbaren Befeuchter umfasst, wobei die Steuereinheit (CU), die in dem Verarbeitungsmodus betrieben wird, dazu konfiguriert ist, den steuerbaren Befeuchter unter Verwendung einer Eingabe, die von dem Feuchtigkeitssensor erhalten wird, dazu zu steuern, eine Zielfeuchtigkeit der Umgebung innerhalb der Verarbeitungskammer (100) aufrechtzuerhalten.

11. System nach einem der Ansprüche 9-10, wobei die Steuereinheit (CU) kommunikativ mit einem Kommunikationsnetz gekoppelt und ferner dazu konfiguriert ist, einen Status des Systems über das Kommunikationsnetz an einen oder mehrere Knoten zu übertragen und/oder über das Kommunikationsnetz Befehle von dem einen oder den mehreren Knoten zu erhalten.

12. System nach einem der Ansprüche 9-11, wobei der Einlass (110) in einer unteren Hälfte der Verarbeitungskammer (100) angeordnet ist.

13. System nach einem der Ansprüche 9-12, ferner umfassend eine Shredder-Baugruppe (700), die fluidisch mit dem Einlass (110) der Verarbeitungskammer (100) gekoppelt ist, die dazu konfiguriert ist, medizinischen Abfall zu erhalten und zu Partikeln medizinischen Abfalls zu verarbeiten und die verarbeiteten Partikel medizinischen Abfalls dem Einlass (110) der Abfallverarbeitungskammer (100) zuzuführen.

14. System nach einem der Ansprüche 9-13, ferner umfassend eine Evakuierungs-Baugruppe (800), die fluidisch mit dem Auslass (120) der Verarbeitungskammer (100) gekoppelt ist, die dazu konfiguriert ist, die verarbeiteten/bearbeiteten Partikel medizinischen Abfalls von der Verarbeitungskammer (100) zu extrahieren und die extrahierten Partikel medizinischen Abfalls zu einem Lagercontainer (850) zu bewegen.

15. Verfahren, das von einer Steuereinheit (CU) eines Systems durchgeführt wird, das eine Abfallverarbeitungskammer (100) nach Anspruch 1 umfasst, wobei die Abfallverarbeitungskammer (100) dazu konfiguriert ist, in einem beliebigen eines Belademodus, eines Verarbeitungsmodus und eines Entlademodus betrieben zu werden, wobei der eine oder die mehreren Sensoren (S1, S2) mindestens einen Temperatursensor umfassen, der dazu konfiguriert ist, eine Temperatur der Umgebung innerhalb der Verarbeitungskammer (100) zu messen, wobei das System ferner eine steuerbare Klimasteuereinheit umfasst, die dazu konfiguriert ist, eine Zieltemperatur der Umgebung innerhalb der Verarbeitungskammer (100) aufrechtzuerhalten, wobei das Verfahren Folgendes umfasst:
Betreiben in dem Belademodus durch:
Öffnen eines Einlasses (110), in dem Belademodus, um die Partikel medizinischen Abfalls zu erhalten,
Schließen eines Auslasses (120), in dem Belademodus, um eine hermetische Dichtung zwischen einer Umgebung innerhalb und einer Umgebung außerhalb der Verarbeitungskammer (100) bereitzustellen,
Rühren, in dem Belademodus, der erhaltenen Partikel medizinischen Abfalls, Betreiben in dem Verarbeitungsmodus durch:
Schließen des Einlasses (110), in dem Verarbeitungsmodus, um eine hermetische Dichtung zwischen einer Umgebung innerhalb und einer Umgebung außerhalb der Verarbeitungskammer (100) bereitzustellen,
Rühren, in dem Verarbeitungsmodus, der erhaltenen Partikel medizinischen Abfalls,
Erzeugen, in dem Verarbeitungsmodus, von Ozon und Aufrechterhalten eines Zielkonzentrationsniveaus von Ozon der Umgebung innerhalb der Verarbeitungskammer (100),
Betreiben in dem Entlademodus durch:
Öffnen des Auslasses (120), in dem Entlademodus, um die Partikel medizinischen Abfalls von der Verarbeitungskammer (100) zu extrahieren,
wobei das Verfahren ferner Aufrechterhalten der Zieltemperatur unter Verwendung der gemessenen Temperatur umfasst.

16. Verfahren nach Anspruch 15, wobei der eine oder die mehreren Sensoren (S1, S2) mindestens einen Ozonsensor umfassen, der dazu konfiguriert ist, ein Ozonkonzentrationsniveau der Umgebung innerhalb der Verarbeitungskammer (100) zu messen, wobei Erzeugen des Ozons und Aufrechterhalten eines Zielkonzentrationsniveaus von Ozon unter Verwendung des gemessenen Ozonkonzentrationsniveaus durchgeführt wird.

17. Verfahren nach einem der Ansprüche 15-16, wobei der eine oder die mehreren Sensoren (S1, S2) mindestens einen Feuchtigkeitssensor umfassen, der dazu konfiguriert ist, die Feuchtigkeit der Umgebung innerhalb der Verarbeitungskammer (100) zu messen, wobei das System ferner einen steuerbaren Befeuchter umfasst, der dazu konfiguriert ist, eine Zielfeuchtigkeit der Umgebung innerhalb der Verarbeitungskammer (100) aufrechtzuerhalten, wobei das Verfahren ferner Aufrechterhalten der Zielfeuchtigkeit unter Verwendung der gemessenen Feuchtigkeit umfasst.

18. Verfahren nach einem der vorhergehenden Ansprüche, wobei:
Betreiben in dem Entlademodus ferner Folgendes umfasst:
Öffnen eines steuerbaren Gaseinlasses, der dazu konfiguriert ist, frische Luft an die Verarbeitungskammer (100) bereitzustellen, und Öffnen eines steuerbaren Gasauslasses, der dazu konfiguriert ist, Gas aus der Umgebung in der Verarbeitungskammer (100) abzulassen.

## Revendications

1. Chambre de traitement des déchets (100) configurée pour fonctionner selon l'un quelconque parmi un mode de chargement, un mode de traitement ou un mode de déchargement, configurée en outre pour fournir, en mode de traitement, un environnement pour désinfecter des particules de déchets médicaux en utilisant de l'ozone, la chambre de traitement comprenant :
une admission (110) configurée, en mode de chargement, pour recevoir les particules de déchets médicaux et configurée, en modes de traitement et de déchargement, pour assurer une étanchéité hermétique entre l'environnement à l'intérieur et un environnement à l'extérieur de la chambre de traitement (100),
une sortie (120) configurée, en mode de déchargement, pour extraire les particules de déchets médicaux hors de la chambre de traitement (100) et configurée, en modes de chargement et traitement, pour assurer une étanchéité hermétique entre l'environnement à l'intérieur et l'environnement à l'extérieur de la chambre de traitement (100) ;
un dispositif de transport des particules (130) configuré, en modes de chargement et de traitement, pour agiter les particules de déchets médicaux et configuré en outre, en mode de déchargement, pour les acheminer vers la sortie (120) pour qu'elles soient extraites,
un ou plusieurs capteurs (S1, S2) configurés pour mesurer des caractéristiques de l'environnement à l'intérieur de la chambre de traitement (100), dans laquelle les un ou plusieurs capteurs (S1 , S2) comprennent au moins un capteur de température configuré pour mesurer la température de l'environnement à l'intérieur de la chambre de traitement (100),
dans laquelle la chambre de traitement (100) est reliée en outre par fluide à ou comprend un générateur (G) configuré pour générer de l'ozone et pour maintenir un niveau de concentration cible d'ozone de l'environnement à l'intérieur de la chambre de traitement (100), dans laquelle la chambre de traitement comprend en outre une unité de climatisation contrôlable configurée pour maintenir une température cible de l'environnement à l'intérieur de la chambre de traitement (100).

2. Chambre de traitement des déchets selon la revendication 1, dans laquelle les un ou plusieurs capteurs (S1, S2) comprennent au moins un capteur d'ozone configuré pour mesurer un niveau de concentration d'ozone de l'environnement à l'intérieur de la chambre de traitement (100).

3. Chambre de traitement des déchets selon l'une quelconque des revendications précédentes, dans laquelle le générateur (G) est fixé de façon amovible à la chambre de traitement (100).

4. Chambre de traitement selon l'une quelconque des revendications précédentes, dans laquelle les un ou plusieurs capteurs (S1, S2) comprennent au moins un capteur d'humidité configuré pour mesurer l'humidité de l'environnement à l'intérieur de la chambre de traitement (100).

5. Chambre de traitement selon l'une quelconque des revendications précédentes, comprenant en outre un humidificateur contrôlable configurée pour maintenir une humidité cible de l'environnement à l'intérieur de la chambre de traitement (100).

6. Chambre de traitement selon l'une quelconque des revendications précédentes, dans laquelle la chambre de traitement (100) est munie de fenêtres d'inspection disposées sur une moitié supérieure de la chambre de traitement (100).

7. Chambre de traitement des déchets selon l'une quelconque des revendications précédentes, dans laquelle le dispositif de transport (130) est muni d'un arbre équipé d'un axe longitudinal parallèle à un axe longitudinal de la chambre de traitement (100), et de palettes s'étendant dans des directions orthogonales à l'axe longitudinal de l'arbre.

8. Chambre de traitement des déchets selon l'une quelconque des revendications précédentes, dans laquelle la chambre de traitement comprend en outre :
une admission de gaz contrôlable configurée pour fournir de l'air frais à la chambre de traitement (100),
un échappement de gaz contrôlable configuré pour évacuer les gaz hors de l'environnement de la chambre de traitement (100).

9. Système de traitement des déchets configuré pour désinfecter des particules de déchets médicaux en utilisant de l'ozone, le système comprenant :
la chambre de traitement des déchets (100) selon la revendication 1,
une unité de commande (CU) configurée pour commander la chambre de traitement (100) pour qu'elle fonctionne selon l'un quelconque parmi un mode de chargement, un mode de traitement ou un mode de déchargement, dans lequel l'unité de commande (CU), lorsqu'elle fonctionne en mode de traitement, est configurée en outre pour commander le générateur (G) en utilisant une entrée reçue en provenance d'au moins un des capteurs (S2, S2), l'entrée étant indicative d'un niveau de concentration cible d'ozone de l'environnement à l'intérieur de la chambre de traitement (100), dans lequel les un ou plusieurs capteurs (S2 , S2) comprennent au moins un capteur de température et le système comprend en outre une unité de commande de climatisation contrôlable, dans lequel l'unité de commande (CU), lorsqu'elle fonctionne en mode de traitement, est configurée pour commander l'unité de commande de climatisation contrôlable en utilisant une entrée reçue en provenance du capteur de température pour maintenir une température cible de l'environnement à l'intérieur de la chambre de traitement (100).

10. Système selon la revendication 9, dans lequel les un ou plusieurs capteurs (S2, S2) comprennent au moins un capteur d'humidité et le système comprend en outre un humidificateur contrôlable, dans lequel l'unité de commande (CU), lorsqu'elle fonctionne en mode de traitement, est configurée pour commander l'humidificateur contrôlable en utilisant une entrée reçue en provenance du capteur d'humidité pour maintenir une humidité cible de l'environnement à l'intérieur de la chambre de traitement (100).

11. Système selon l'une quelconque des revendications 9 à 10, dans lequel l'unité de commande (CU) est reliée en communication à un réseau de communication et configurée en outre pour transmettre l'état du système à un ou plusieurs nœuds par l'intermédiaire du réseau de communication et/ou recevoir des commandes en provenance des un ou plusieurs nœuds par l'intermédiaire du réseau de communication.

12. Système selon l'une quelconque des revendications 9 à 11, dans lequel l'admission (110) est disposée sur une moitié inférieure de la chambre de traitement (100).

13. Système selon l'une quelconque des revendications 9 à 12, comprenant en outre un ensemble de broyage (700) relié par fluide à l'admission (110) de la chambre de traitement (100), lequel est configuré pour recevoir des déchets médicaux et les traiter en particules de déchets médicaux, et pour alimenter l'admission (110) de la chambre de traitement des déchets (100) avec les particules de déchets médicaux traitées.

14. Système l'une quelconque des revendications 9 à 13, comprenant en outre un ensemble d'évacuation (800) relié par fluide à la sortie (120) de la chambre de traitement (100), lequel est configuré pour extraire les particules traitées de déchets médicaux de la chambre de traitement (100) et pour déplacer les particules extraites de déchets médicaux vers un conteneur de stockage (850).

15. Procédé mis en œuvre par une unité de commande (CU) d'un système comprenant une chambre de traitement des déchets (100) selon la revendication 1, la chambre de traitement des déchets (100) étant configurée pour fonctionner selon l'un quelconque parmi un mode de chargement, un mode de traitement et un mode de déchargement, dans lequel les un ou plusieurs capteurs (S1 , S2) comprennent au moins un capteur de température configuré pour mesurer la température de l'environnement à l'intérieur de la chambre de traitement (100), dans lequel le système comprend en outre une unité de commande de climatisation contrôlable configurée pour maintenir une température cible de l'environnement à l'intérieur de la chambre de traitement (100), le procédé comprenant :
le fonctionnement en mode de chargement par :
l'ouverture d'une admission (110), en mode de chargement, pour recevoir les particules de déchets médicaux,
la fermeture d'une sortie (120), en mode de chargement, pour assurer une étanchéité hermétique entre un environnement à l'intérieur un environnement à l'extérieur de la chambre de traitement (100),
l'agitation, en mode de chargement, des particules de déchets médicaux reçues,
le fonctionnement en mode de traitement par :
la fermeture de l'admission (110), en mode de traitement, pour assurer une étanchéité hermétique entre un environnement à l'intérieur et un environnement à l'extérieur de la chambre de traitement (100),
l'agitation, en mode de traitement, des particules de déchets médicaux reçues,
la génération, en mode de traitement, d'ozone et le maintien à un niveau de concentration cible de l'ozone dans l'environnement à l'intérieur de la chambre de traitement (100),
le fonctionnement en mode de déchargement par :
l'ouverture de la sortie (120), en mode de déchargement, pour extraire les particules de déchets médicaux hors de la chambre de traitement (100),
dans lequel le procédé comprend en outre le maintien de la température cible en utilisant la température mesurée.

16. Procédé selon la revendication 15, dans lequel les un ou plusieurs capteurs (S1, S2) comprennent au moins un capteur d'ozone configuré pour mesurer un niveau de concentration d'ozone de l'environnement à l'intérieur de la chambre de traitement (100), dans lequel la génération d'ozone et le maintien d'un niveau de concentration cible d'ozone sont effectués en utilisant le niveau de concentration d'ozone mesuré.

17. Procédé selon l'une quelconque des revendications 15 à 16, dans lequel les un ou plusieurs capteurs (S1, S2) comprennent au moins un capteur d'humidité configuré pour mesurer l'humidité de l'environnement à l'intérieur de la chambre de traitement (100), dans lequel le système comprend en outre un humidificateur contrôlable configuré pour maintenir une humidité cible de l'environnement à l'intérieur de la chambre de traitement (100), dans lequel le procédé comprend en outre le maintien de l'humidité cible en utilisant l'humidité mesurée.

18. Procédé selon l'une quelconque des revendications précédentes, dans lequel :
le fonctionnement en mode de déchargement comprend en outre :
l'ouverture d'une admission de gaz contrôlable configurée pour fournir de l'air frais à la chambre de traitement (100) et l'ouverture d'une sortie de gaz contrôlable configurée pour évacuer le gaz hors de l'environnement dans la chambre de traitement (100).
